# EUROPEAN PATENT SPECIFICATION

(11) **EP 4 515 227 B1**
(45) Date of publication and mention of the grant of the patent: **15.07.2026**
(21) Application number: 23721788.0
(22) Date of filing: 28.04.2023
(51) Int. Cl.: G01N 33/18, G01N 33/24

(54) **NETWORK AND METHOD FOR THE MONITORING OF SOIL AND/OR SURFACE WATER QUALITY**
NETZWERK UND VERFAHREN ZUR ÜBERWACHUNG DER BODEN- UND/ODER OBERFLÄCHENWASSERQUALITÄT
RÉSEAU ET PROCÉDÉ DE SURVEILLANCE DE LA QUALITÉ DES SOLS ET/OU DES EAUX DE SURFACE

(30) Priority: 29.04.2022 NL 1044306; 12.03.2023 NL 1044564
(43) Date of publication of application: 05.03.2025
(73) Proprietor: EasyMeasure Developments B.V., 8413 NN Oudehorne (NL)
(72) Inventor: MAYER, Mateo Jozef Jacques, 3811 BJ AMERSFOORT (NL); AKKERMAN, Franke, 3811 BJ AMERSFOORT (NL); OUDAKKER, Michiel, 3811 BJ AMERSFOORT (NL); WAGTERVELD, René Martijn, 3811 BJ AMERSFOORT (NL)
(86) International application number: PCT/NL2023/050228
(87) International publication number: WO 2023/211277

(56) References cited:
- CN-A- 106 706 876
- CN-A- 112 798 333
- CN-U- 204 649 234
- KR-B1- 102 187 336
- US-A1- 2020 364 456
- US-A1- 2021 140 908

## Description

The present invention relates to a network of sensors for measuring soil and/or surface water quality, a method for measuring soil and/or surface water quality and a computer program to carry out the method. The invention further relates to the use of the network for measuring the robustness of agricultural land against droughts and heavy rains and/or for measuring and/or calculating an optimal nutrient concentration in agricultural land and/or for determining the presence or absence of a correlation between a quality of agricultural land and the wash-out of nutrients to surface water.

The surface water in our living environment is vital for our health and wellbeing and provides essential habitat for various plants and animals. We use our surface water for recreation, such as swimming, sailing, and drinking water production. Unfortunately, the quality of our surface water is under continuous pressure, mainly because of our use of medicines, pesticides and plastics.

The primary source of medicines in our surface water originates from flushing our toilets. The toilet water ends up in municipal wastewater treatment plants (MWTTPs), where the treatment plant biologically purifies the water before being discharged to the surface water. Unfortunately, the biological process at MWTTPs is not sufficiently efficient to remove all medicine traces. As a result, the MWTTPs release many medicines to the surface water through the effluent.

The primary source of pesticides in our surface water is agriculture for crop protection. Traces of these pesticides are extracted from the soil by rainwater and transported to the surface water.

Our use of plastics and the resulting plastic waste in our environment results in microplastics in surface water. Microplastics are small plastic particles originating from eroded and partially decomposed plastic waste.

Medicine traces, pesticides and plastics are often referred to as micropollutants since their presence is in the range of micrograms or even nanograms per litre of water.

Micropollutants may seriously affect the metabolism of humans and other living organisms since a number of these components and their metabolites appear to interfere with, amongst others, hormone balances.

Past two decades, it became clear that we need to pay more attention to improving the quality of our surface water since the micropollutants in our surface water are only partially removed during the drinking water production process. As a result, increasing levels of micropollutants affect our health.

The importance of suitable surface water quality is internationally recognized. In 2000, the EU adopted the so-called EU Water Framework Directive (WFD). The WFD comprises a timetable for the stepwise realization of water quality improvement steps to be executed by 2033.

Besides micropollutants, high nutrient concentrations in the water, such as ammonium ions, phosphate ions and nitrates, may seriously affect water quality. These components originate from manure and fertilizer used in agriculture and other organic waste materials ending up in our surface water during periods of rain.

A high concentration of nutrients in surface water results in excessive growth of algae and cyanobacteria, often referred to as blue-green algae. As a result, the biological equilibrium in the water is disturbed, which may result in the excessive dying of fish and other aquatic organisms due to periods of low oxygen concentration in the water. Additionally, the bluegreen algae may produce toxins and taste-and-odour compounds that may cause serious public health concerns and substantial economic damage in recreation areas since people cannot recreate in the water during periods of algae bloom.

To summarize, it is essential for our health, wellbeing and environment that we safeguard the quality of our surface water. Important points of attention are the micropollutants and large amounts of nutrients that are currently accumulating in our environment and surface water due to our human footprint.

KR 102 187 336 B1 discloses a water quality monitoring system in which a security function is reinforced by blockchain application and communication safety is ensured.

At present, governments monitor surface water quality periodically at a limited number of public swimming places, especially during the swimming season. In the EU, a directive for monitoring swimming water quality i.e., Directive 76/160/EEC, is in force since February 2006. In a nutshell, this directive describes how to ensure public health by periodic sampling and analyzing public surface water and by determining the concentration of Escherichia coli and Intestinal enterococci in water as well as the risk of cyanobacterial (green-blue algae) bloom.

An important drawback of the present monitoring procedure is the relatively high labor cost for taking samples, preparing the samples for analysis and performing the analyses. Because of the high cost involved, monitoring is taking place at only a limited number of public swimming places and at a relatively low frequency. As a consequence, undesired changes in water quality are often not detected, or detected too late.

The present invention aims to obviate or at least reduce the abovementioned problems. To that end, the invention provides a network of sensors for measuring soil and/or surface water quality according to claim 1

With the technology according to the present invention we aim to solve this problem by providing a community-driven monitoring system to monitor the soil and/or surface water quality in our living environment. The network according to the invention provides the advantage that real-time monitoring of soil and/or surface water becomes possible in a cost-effective manner. In addition, the network allows the quality of both soil and surface water to be predicted using the information that is gathered by and in the network.

The method and devices according to the present invention will make it possible to monitor surface water quality real-time and inline at a very large number of places in a very cost effective way. Changes in water quality will be detected by the network at a very fast rate. At the same time, it will also be possible to make water quality predictions based on historical sensor data, historical weather data and the weather forecast. This approach opens possibilities for water quality labs to selectively increase the sampling in regions with high risks for bad water quality and, if necessary, to warn the government with an advice to take action.

Hence, the method and devices according to the present invention will be an important tool and call to action for the community and the government to jointly improve the surface water quality in our living environment.

It is noted that in a preferred embodiment, the soil data is soil moisture data and the soil sensor is a soil moisture data.

It will be understood that the algorithm(s) used in the method involve several steps, preferably including system identification. Preferably, the identification involves creating/improving first-principles models to estimate parameters of interest. **In** addition, in case the identification identifies missing parts, such as non-observable/detectable elements, these parts are preferably covered by developing data driven machine learning algorithm to fill the gaps of the missing parts. This preferably results in the creation of a so-called hybrid white/black box model, combining first-principles with data driven machine learning algorithms. It will be understood that in these steps use can be made of existing modelling parts and modelling techniques.

In presently preferred embodiments of the invention data from sensors provide on-line, real-time input for the model prediction, and can be used to improve the model prediction. As the spectra combine information of several components present in e.g. surface or drinking water, artificial intelligence can be used to increase sensitivity and selectivity. The presently preferred overall modelling approach is based on a first-principles model with an (AI) machine learning algorithm. The addition of a first-principles model strongly increases the accuracy of the estimators and is therefore relevant for robust modelling. First-principles models can for example be created based on a nonlinear, reduced-order state-space model, while data-driven models can for example be based on multiple linear regression, K nearest neighbors, gradient-boosting decision tree, and support vector regression. Advanced artificial intelligence (AI) and geospatial probabilistic risk modeling can be used to choose the best locations for monitoring water quality.

In presently preferred embodiments of the invention the information collected by sensors is used in real-time to make predictions using a model. This data can also be used to improve the model's accuracy. For example, the spectra of drinking water contains information about multiple components, which can be analyzed using artificial intelligence to increase sensitivity and selectivity. The most effective approach is to use a first-principles model with a machine learning algorithm, which significantly improves the accuracy of the estimators and is essential for robust modeling.

In an embodiment according to the present invention further relates to a method, network of devices, network of data and network of sensor fusion algorithms for the monitoring of surface water quality, characterized by at least a first sensor from the group of soil moisture sensors with at least two conductive pins, both operatively connected to soil, at least a second sensor from the group of floating water quality sensors, operatively connected to surface water, means to transfer the sensor data to at least a first server and a first blockchain, a first or soil sensor fusion algorithm translating soil moisture and surface water sensor data to a present and future surface water quality, a water sensor fusion algorithm translating the water quality data to a surface water finger print, a first blockchain algorithm that automatically sends tokens existing on the first blockchain to sensor owners as a reward for uploading sensor data to said first server and said first blockchain and a publicly available map with an overview of the sensor locations and the surface water quality data so that public awareness is created on surface water quality.

The method and network of devices according to the present invention make it possible to improve the quality of the surface water in our living environment through a community driven ecosystem in which people are empowered to monitor and measure their local water quality and are rewarded with crypto tokens for contributing water quality data to the network.

In an embodiment of the network according to the invention, the at least one sensor from the group soil sensors is a soil moisture sensor that is configured for measuring at least soil resistance between at least two electrodes, air temperature, relative humidity of the air, air pressure and VOC content in the air.

An advantage of the soil moisture sensor is that it measures the impedance of the soil between both electrodes which contains information on the moisture content in the soil and the concentration of nutrients in the soil. The combination with other parameters allows the concentration of nutrients in the soil moisture and the soil moisture content to be obtained in a more detailed manner.

In an embodiment of the network according to the invention, the at least one sensor from the group of water quality sensors is a floating water quality sensor that is further configured for measuring at least water temperature, transmission of light through the water at five or more wavelengths in the UVA and/or visible and/or nIR regions.

It was found that water temperatures provides important information on the resulting increased risk for bacterial and algal growth rates. An increase in electrical conductivity of the water appears to indicate an increased concentration of nutrients in the water as well as an increased risk for bacterial and algal growth. Finally, specific changes in the light transmission profile of the spectrophotometric sensors indicate both an increased concentration of green algae or blue green algae (cyanobacteria) and an increased concentration of nutrients in the surface water. Therefore, this combination is especially useful in the network to monitor water quality of the surface water.

In an embodiment of the network according to the invention, the parameter P_{wash} is a "local wash out of nutrients from the soil" parameter P_{wash}.

In an embodiment of the network according to the invention, the at least one water sensor fusion algorithm is configured to translate the water quality sensor data into a concentration of green algae and/or blue-green algae in the surface water.

An advantage is that blue-green algae can be detected in a more detailed and effective manner. This is especially relevant since blue-green algae produce harmful toxines, thus making the blue-green algae concentration in the water an important swimming water quality parameter.

In an embodiment of the network according to the invention, the soil moisture sensors comprise pinners, and wherein the water quality sensors comprise type one floaters and type two floaters.

Preferably, two types of floaters (floating water quality sensors) are used. Both are configured to measure temperature and water conductivity. In addition, type one is preferably configured to measure light transmission at 5 wavelengths in the UVA and visible regions and/or near infrared (nIR) regions. The second type is preferably configured to measure 7 wavelengths and the fluorescence spectrum resulting from excitation wavelengths at each of these 7 wavelengths in the range between 300 nm and 850 nm, and more preferably with a resolution of 300 measuring points.

In an embodiment of the network according to the invention, the network may further comprise:
- at least one graphical representation of sensor data on a map with GPS coordinates characterized by means to express both the overall surface water quality as well as means to characterize the certainty of the surface water quality assessment;
- a reward system that automatically rewards sensor owners with crypto tokens relative to the amount of data packets their sensors upload to the network of surface water quality sensors.

The advantage of providing a reward system that automatically rewards sensor owners with crypto tokens relative to the amount of data packets their sensors upload to the network is that it increases the number of users that provide data to the network. Since data is important for the network to be operated in an efficient and effective manner, this reward system may increase the effectiveness of the system. A non-limiting example of a blockchain that is feasible for such reward system is the Algorand blockchain.

The advantage of providing data on a map is that users can readily spot and interpret the data that is available in the system. This increases user friendliness and, in case of swimming water, increases safety since users can immediately see whether the water is safe for swimming or not.

In an embodiment of the network according to the invention, the individual sensor data stored in the blockchain is a hash of the sensor data received from the sensors and/or the individual sensor data stored by the server.

The server and blockchain make the data readily accessible for a multitude of users and allows the data to be collected in a central location. An additional advantage of blockchain is that the data is fixed after first storage, thus preventing any changes and/or tampering.

In an embodiment of the network according to the invention, both a type one and a type two floating water quality sensor is configured to measure a temperature of the water, electrical conductivity (EC) of the water, and wherein:
- a type one floating water quality sensor is further configured to measure light transmission at preferably 5 wavelengths in the UVA and visible regions and/or near infrared (nIR) regions; and
- a type two floating water quality sensor is further configured to measure light transmission at preferably 7 wavelengths and the fluorescence spectrum resulting from excitation wavelengths at each of these 7 wavelengths in the range between 300 nm and 850 nm,
and wherein the network comprises at least one sensor from the group of type two floating water quality sensors.

The advantage of providing two different sensor types is that each provides an additional advantage of operating at different wavelengths, thus leading to an increase in the available data and the quality of that data.

In an embodiment of the network according to the invention, the network may further comprise at least one VOC sensor that is configured to measure a VOC-content in the air, and wherein the at least one VOC sensor is positioned at a predetermined distance from a ground surface and preferably is positioned at a distance of at most 1 meter above the ground surface.

An advantage of providing a VOC sensor is that information on the biological activity in the soil and on the soil surface can be provided. This biological activity is related to the TVOC concentration in the soil, which is an important parameter in soil quality optimization. It is also noted that TVOC is not only related to soil quality in terms of robustness against changes in weather conditions, such as drought or heavy rainfall but also to the effectiveness of the soil to biologically decompose pesticides that were used for crop protection.

In addition, or as an alternative to the VOC sensor, other sensors can be included in further preferred embodiments of the invention, such as an NOx sensor and/or ammonium sensor and/or CH4 sensor. Preferably, these measurements are performed in air at a presently preferred height of some centimeters above the ground surface. A further example of an additional or alternative sensor for the soil and/or water quality is a sensor that applies so-called mid infrared technology, using a midinfrared source preferably operating in the range between 2 µm to 12 µm, and in this wavelength range measures components like H2O, CO2, CO, NO, NO2, CH4, O3, and/or NH3.

In an embodiment of the network according to the invention, the network may further comprise one or more sensors configured to measure weather conditions, preferably local weather conditions, and wherein the weather data preferably comprise one or more of air humidity, air pressure, and/or (air) temperature.

An advantage of providing additional sensors for different parameters is that the quality of the data is improved, leading to a more detailed and specific assessment of the soil and/or water quality.

An advantage of providing at least one controller and/or processor is that the network is capable of performing the necessary data processing and/or calculations, including for example the sensor fusion algorithms. Another advantage is that this increases reliability and flexibility of the network, thus making it more effective.

In an embodiment of the network according to the invention, the network, preferably the controller and/or processor and/or the sensor fusion algorithm, are configured to process weather data and/or weather prediction data.

The weather data is preferably processed by either the sensor fusion algorithm or the processor into useful data that is sent and stored in the network.

In an embodiment of the network according to the invention, the network, preferably one or more of the sensor fusion algorithms therein, is configured to calculate a present and/or future quality of agricultural land expressed in conformity parameters Dm and Dt over time.

An advantage of the conformity parameters is that it allows an assessment of water and/or soil quality at a glance. This improves monitoring, control and legal compliance for the users of the data, such as farmers and government bodies.

In addition, it enhances the control system and provides tools to more clearly and realistically control the environment while maintaining the possibility for economically feasible agricultural production.

The invention also enables providing a system for measuring soil and/or surface water quality, the system comprising:
- a network according to the invention;
- at least one computing device configured to perform one or more of the at least one soil sensor fusion algorithm, the at least one water sensor fusion algorithm and the at least one conformity sensor fusion algorithm that is configured for producing data conformity parameters Dm and Dt; and
- preferably one or more user interfaces allowing users of the system to provide data to the system and/or to extract data from the system and/or to modify data in the system, wherein the modifying is preferably at least based on sensor data provided by the network.

The system provides similar effects and advantages as described for the network according to the invention. The system may freely be combined with one or more of the embodiments as described for the network according to the invention.

The invention also relates to a method according to claim 8 for measuring soil and/or surface water quality.

The method according to the invention provides similar effects and advantages as described for the network and the system according to the invention. The method according to the invention may freely be combined with one or more of the embodiments as described for the network or the system according to the invention.

In particular, the method allows community-driven monitoring to monitor the soil and/or surface water quality in our living environment by providing real-time monitoring of soil and/or surface water in a cost-effective manner. In addition, the method allows the quality of both soil and surface water to be predicted using the information that is gathered by and in the network.

The method also allows surface water quality and soil quality to be monitored real-time, inline at a very large number of places in a very cost effective way. Another advantage is that the method allows changes in water quality to be detected in a rapid and effective manner. A further advantage of the method according to the invention is that water quality predictions can be made based on historical sensor data, historical weather data and the weather forecast. This approach opens possibilities for water quality labs to selectively increase the sampling in regions with high risks for bad water quality and, if necessary, to warn the government with an advice to take action.

In an embodiment of the method according to the invention, the method may further comprise the steps of:
- sending the sensor data to a server and/or blockchain;
- storing the sensor data in the blockchain and/or at the server, preferably as individual sensor data containing time stamps of the data, the sensor data and the GPS coordinates of each sensor.

Sending and storing the data in a central location allows the data to be accessed in a simple and effective manner. In addition, by including time stamps and GPS coordinates, the data can easily be used in the network and for other purposes relating to water and/or soil quality.

In an embodiment of the method according to the invention, the method may further comprise the steps of:
producing, using at least one conformity sensor fusion algorithm, data conformity parameters Dm and Dt for each sensor in at least one region G,
wherein the producing comprises providing a measure for the momentary and time averaged deviations from sensor data to all sensor data in region G respectively.

This embodiment provides a more integrated overview of water and/or soil quality in an accessible manner, thus allowing other users to access and use the data more easily. Another advantage is that the conformity parameters show, preferably in the blink of an eye, the status of water quality in a specific location or region. This is for example highly useful in monitoring water quality in locations for swimming.

In an embodiment of the method according to the invention, the method is a computer-implemented method.

An advantage of providing the method as a computer-implemented method is that it can be rapidly expanded due to for example its scalability. In addition, it provides an improved accessibility.

The invention also relates to a computer program comprising instructions which, when the program is executed by a computer, cause the computer to carry out the steps of the method according to the invention.

The computer program according to the invention provides similar effects and advantages as described for the network, the system and the method according to the invention. The computer program according to the invention may freely be combined with one or more of the embodiments as described for the network, the system and/or the method according to the invention.
- The invention also relates to a method according to claim 11 for realizing a network of surface water quality sensors according to the invention.

The method for realizing a network of surface water quality sensors according to the invention provides similar effects and advantages as described for the network, the system and the method according to the invention. The method for realizing a network of surface water quality sensors according to the invention may freely be combined with one or more of the embodiments as described for the network, the system or the method according to the invention.

The invention further relates to a method for measuring the robustness of agricultural land against droughts and heavy rains by using a network according to the invention.

The method for measuring robustness according to the invention provides similar effects and advantages as described for the network and the system according to the invention. The method for measuring robustness according to the invention may freely be combined with one or more of the embodiments as described for the network or the system according to the invention.

The invention further relates to a method for measuring and/or calculating an optimal nutrient concentration in agricultural land by using a network according to the invention.

The invention further relates to a method for measuring and/or calculating an optimal nutrient concentration according to the invention provides similar effects and advantages as described for the network and the system according to the invention. The method for measuring and/or calculating an optimal nutrient concentration according to the invention may freely be combined with one or more of the embodiments as described for the network or the system according to the invention.

The invention further relates to a method for determining the presence or absence of a correlation between a quality of agricultural land and the wash-out of nutrients to surface water by using a network according to one embodiment of the invention.

The method for determining correlation according to the invention provides similar effects and advantages as described for the network and the system according to the invention. The method for determining correlation according to the invention may freely be combined with one or more of the embodiments as described for the network or the system according to the invention.

The invention further relates to the use of a network according to the invention for measuring the robustness of agricultural land against droughts and heavy rains.

The network may advantageously be used to provide a more detailed insight in the robustness of an agricultural region or piece of land. The insight gained might be used to provide evidence with regard to legal compliance. Alternatively, or additionally, it may also be used to make (more) detailed mitigation plans to mitigate the effects of climate change on the agriculture.

The invention further relates to the use of a network according to the invention for measuring and/or calculating an optimal nutrient concentration in agricultural land.

The network may advantageously be used to provide evidence with regard to legal compliance, especially with regard to nutrient disposition on the land. Alternatively, or additionally, it may also be used to make (more) detailed mitigation plans to mitigate the effects of climate change on the agriculture.

The invention further relates to the use of a network according to the invention for determining the presence or absence of a correlation between a quality of agricultural land and the wash-out of nutrients to surface water.

By providing a more detailed insight, the balance between the requirements of nature (repair) and the requirements of agriculture can be more easily be achieved and/or maintained.

The abovementioned uses of the network according to the invention provide similar effects and advantages as described for the network and the system according to the invention. These uses may freely be combined with one or more of the embodiments as described for the network or the system according to the invention.

According to an embodiment of the network, the present invention relates to at least one sensor from the group of pinners i.e., soil moisture sensors. Preferably, a soil moisture sensor is equipped with at least 2 electrode pins made of stainless steel or titanium which are placed in the ground. Preferably, the electrodes have a length of at least 20 cm and are placed in the ground at a distance of about 5 cm from each other. The pins are operatively connected to an AC source with a frequency of preferably 1 kHz. This frequency can easily be generated by the PWM (pulsed width modulation) output of a first microcontroller, a NPN transistor as amplifier and a coupling capacitor to obtain an AC source. Preferably, the impedance of the wet soil between the electrodes is measured by the use of an analog to digital converter which is preferably connected to the first microcontroller. It is noted that the impedance of the soil between the electrodes depends on the moisture in the soil and on the conductivity of the moisture in the soil. The conductivity of the moisture in the soil appears to be a good measure for the concentration of nutrients that are present in the soil. Summarizing, the soil moisture sensor measures the impedance of the soil between both electrodes which contains information on the moisture content in the soil and the concentration of nutrients in the soil. Stand-alone application of the soil moisture sensor according to the present invention brings along several practical problems:
1. It is not possible to discriminate between a high moisture content with low conductivity and a low moisture content with high conductivity since both moisture contents may result in the same impedance of the soil between the electrodes.
2. It is not possible to discriminate between a high moisture content in the upper part of the soil and a high moisture content in the lower part of the soil, since both moisture contents may result in the same impedance of the soil between the electrodes.

With the method according to the present invention, comprising sensor fusion, it is possible to take away both disadvantages. In order to take away the first disadvantage, the soil sensor is preferably equipped with a air humidity sensor, a temperature sensor and an air pressure sensor. These additional sensors collect data on the weather conditions at the location where the soil sensor is placed in the ground. By recording a history file of weather data together with the soil impedance data and by application of an algorithm with the impedance data and the weather data as input, the inventors of the technology according to the present invention, further on referred to as "the inventors", were able to derive the concentration of nutrients in the soil moisture and the soil moisture content from the soil sensor data. The inventors also found that an additional VOC (volatile organic compounds) sensor, measuring the VOC content in the air close to the ground, adds additional information to the data, making the prediction of the concentration of nutrients in the soil even more accurate.

In a preferred embodiment of a soil sensor according to the present invention, the soil sensor consists of at least 2 electrodes, preferably with a length of at least 20 cm, that are operatively connected to the soil, means to determine the impedance of the soil between the electrodes using an AC source, a temperature sensor measuring the temperature of the air and / or the soil, an air humidity sensor and a VOC sensor measuring the VOC concentration in the air close to the ground, means for storing the sensor data, resulting in a history file of the data and an algorithm translating the sensor data to a soil moisture content in the upper part of the sensor, a soil moisture content in the lower part of the sensor, a nutrient concentration in the soil moisture in the upper part of the sensor and a nutrient concentration in the soil moisture in the lower part of the sensor.

According to an embodiment of the network, the present invention relates to a soil sensor fusion algorithm converting the soil sensor data into a "local wash out of nutrients from the soil parameter" P_{wash}. The value of P_{wash} at a certain location L is related to history file of temperature, air humidity, rainfall, soil humidity in the upper part of the soil, soil humidity in the lower part of the soil, conductivity in the upper part of the soil and conductivity in the lower part of the soil, all at location L. The value of P_{wash}(L) indicates the amount of nutrients that will be washed out from the soil and collect in the surface water near L. The values of P_{wasn}(L) in a certain region combined with a weather forecast for that area make it possible to make a surface water quality forecast at location L. Making surface water quality forecasts based on local P_{wash}(L) values and actual local surface water qualities, expressly makes part of the technology according to the present invention. According to an embodiment of the network, the present invention relates to at least one sensor from the group of floaters i.e., floating water quality sensors. A first type of floating water quality sensors, further on referred to as "type one floaters", preferably measures temperature of the water, electrical conductivity (EC) of the water and light transmission at preferably 5 wavelengths in the UVA and visible regions and / or near infrared (nIR) regions. A second type of floating water quality sensors, i.e., type 2 floaters, measures temperature of the water, EC, light transmission at preferably 7 wavelengths and the fluorescence spectrum resulting from excitation wavelengths at each of these 7 wavelengths in the range between 300 nm and 850 nm, preferably with a resolution of 300 measuring points. The inventors have found that the first type of floaters provide important information on the surface water quality. Water temperatures above 20 degrees Celsius appear to provide important information on the resulting increased risk for bacterial and algal growth rates. An increase in electrical conductivity of the water appears to indicate an increased concentration of nutrients in the water as well as an increased risk for bacterial and algal growth. Finally, specific changes in the light transmission profile of the spectrophotometric sensors indicate both an increased concentration of green algae or blue green algae (cyanobacteria) and an increased concentration of nutrients in the surface water. Especially, an increased absorption of UVA light, blue light and red light as compared to green light are a strong indication for algal growth. An increasing absorption with decreasing wavelength is a strong indication for an increase in the concentration of nutrients in the surface water. The inventors have found that the second type of floaters is able to measure the same information as the first type of floaters. On top of that, the second type of floaters is able to specifically detect green algae and blue-green algae. Since blue-green algae produce harmful toxines, the blue-green algae concentration in the water is an important swimming water quality parameter. Because of this reason, the second type of floaters can be used in addition to the first type of floaters to provide extra information on water quality.

According to an embodiment of the network, the present invention relates to a water sensor fusion algorithm translating the information produced by type one floaters and / or type two floaters to a concentration of nutrients in the surface water and a concentration of green algae and / or blue-green algae in the surface water based on water temperature, water conductivity and the transmission quotients of all transmissions measured with the type one floaters and / or type two floaters. In case type two floaters are applied, the surface water quality data can be further enriched by using the fluorescence spectrum at the different LED wavelengths, i.e, at the different excitation wavelengths, of the type two floaters. The fluorescence spectra produced by the type two floaters can be used to quantitatively determine the concentration of green algae and blue-green algae in the surface water. This means that the type two floater not only provides additional information on the surface water quality, but can also be used to validate the water sensor fusion algorithms translating the pinner and floater type one measurements into surface water quality. It is noted that type two floater can also determine different species of green algae and blue-green algae since these species have different absorption and fluorescence characteristics. Application of the type two floaters to determine the algae species present in the surface water and using the resulting information to improve the sensor fusion algorithms expressly makes part of the present invention.

In addition, or as an alternative to the sensors of the type one floaters and/or type two floaters, in some of the presently preferred embodiments of the invention use is made of photos of the surface water that can be analyzed to determine a measure for water quality. For example, such measure may relate to the surface coverage (SC) degree of the water body with water plants.

According to an embodiment of the network, the present invention relates to first means for connecting the pinners, type one floaters and type two floaters to a server and / or second means for connecting the pinners, type one floaters and type two floaters to a blockchain. The first means and second means for connecting the pinners and floaters to a server and / or blockchain consist of hardware from the groups of 15luetooth connectivity devices, wifi connectivity devices, LoRa connectivity devices, M2M connectivity devices, connectivity devices using mobile technologies like SIM cards, connectivity devices using 5G technology, GPRS devices or connectivity devices using license-free radio communication bands. The server receiving data from the first means for connecting the sensors is equipped with software and hardware to fill and store a database containing time stamps of the data, the sensor data and the GPS coordinates of each sensor.

Optionally, the database is stored on a blockchain so that the data integrity is secured. Preferably, a hash of the data is stored on a blockchain instead of the data. This way, data integrity can be guaranteed without the need to burden the blockchain with large amounts of data. It is also possible to upload the data directly from the sensor into to blockchain using second means for connection. These second means for connection are characterized by the previously mentioned hardware to connect the sensors increased with wallet software and blockchain transaction software in the sensors i.e., in the pinners and floaters of type one and type two. An example of a blockchain that is very feasible for this approach is the Algorand blockchain.

According to an embodiment of the network, the technology of the present invention relates to a sensor fusion algorithm aggregating data from different locations in a user defined geographical region G. The algorithm produces data conformity parameters Dm and Dt for each sensor in region G. The conformity parameters Dm and Dt of a sensor S are a measure for the momentary and time averaged deviations from sensor S data to all sensor data in region G respectively. In other words: high values of Dm and / or Dt of a sensor S indicate that, at the location where sensor S installed, unexpected and / or undesired changes in water quality are taking place.

According to an embodiment of the network, the technology of the present invention relates to a conformity sensor fusion algorithm raising sensor network alarms based on the Dm and / or Dt values of all sensors installed in the network. This approach cannot only be applied within a geographic region G but also to compare the sensor behavior between different geographic regions K and L. The automatic comparison of differences in Dm and Dt behavior between different geographic regions K and L expressly makes part of the present invention.

According to an embodiment of the network, the technology of the present invention relates to a graphical representation of sensor data on a map with GPS coordinates characterized by means to express both the overall surface water quality as well as means to characterize the certainty of the surface water quality assessment. An example of such representation is a colored hexagon symbol on a map with a white circle in the middle of the hexagon: the color indicates water quality e.g., green is good quality and red is bad quality, and the white circle in the hexagon indicates the certainty of the data e.g., a small dot indicates a high certainty and a large dot indicates a low certainty of the data. It is noted that the graphical representation of water quality data on a chart, combining water quality and certainty of the data is essential for a good overview of the water quality in a geographic region. Therefore, it makes expressly part of the technology of the present invention. According to an embodiment of the network, the present invention relates to a reward system for sensor owners to share their data to the network. This reward system automatically rewards sensor owners with crypto tokens relative to the amount of data packets their sensors upload to the network. A non-limiting example of a blockchain that is feasible for such reward system is the Algorand blockchain.

According to an embodiment of the network, the present invention relates to assessing a fingerprint of surface water by the use of the sensors and sensor fusion algorithms defined in this application. With fingerprint, the inventors mean in this application a series of water quality specific signals produced by the sensors combined with a series of water quality parameters that result from the sensor fusion algorithms.

In this section, a number of particularizations and special applications of the technology is described. The claims are based on the description in this section and the description in the detailed introduction as well as the other application text.

It is noted that the description in this section has a strong focus on application of the technology according to the present invention in the market segment of agriculture and farming. This was done to describe the technology of the present invention through concrete examples. Application of the technology in other market segments, such as nature preservation, expressly make part of the technology according to the present invention. Farmers have a license to produce food on agricultural soil and a responsibility towards society to do this in an environmentally responsible way. From a plant nutrients point of view, farming can be seen as an optimization problem: under-fertilization of the soil results in a decrease of food production and over-fertilization results in washout of nutrients like nitrate (total nitrogen) and phosphate (total phosphorous) to the environment. The impact of over-fertilization on our environment is disturbance of ecological equilibrium, both in surface water and on land. In surface water, this may result in excessive plant growth, algal bloom and periods of low oxygen concentrations in water. On land it may result in excessive plant growth of only few plant species and decrease of biodiversity.

Another reason for farmers to optimize their agricultural operations is saving cost and increase production at the same time. A stable and optimum concentration of nutrients in the soil can be achieved through realizing a large concentration of living total organic carbon (LTOC) in the soil. As a result, a relatively large concentration of nutrients is "captured" in the soil and effectively protected against washout. In practice, high concentrations of LTOC in the soil are achieved by preventing periods of under-fertilization and over-fertilization so that there is sufficient time to achieve ecological equilibrium and a high LTOC concentration in the soil. High concentrations of LTOC in the soil also protect the soil against losing to much water during periods of drought thereby increasing crop yield. High LTOC concentrations also decrease the need for soil irrigation since they retain moisture.

In the following, it is explained that the technology of the present invention is a very useful tool for farmers to optimize application of nutrients (fertilizer, green fertilizer or manure) to their soil and achieve maximum LTOC values in the soil, without causing over-fertilization with beforementioned negative impact on our environment. As a result, the technology according to the present invention will help farmers to reduce production cost, maximize crop yield and minimize the effect of their operations on our living environment. Hence, the technology according to the present invention is also a tool for farmers to show and prove responsible care towards the government and society regarding their license to produce.

According to an embodiment of the network, the technology according to the present invention consists of a conductivity sensor operatively connected to the soil, preferably measuring in the soil layer up to a depth of 50 cm. Such conductivity measurement can be conductive or capacitive. As previously explained, the conductivity of the soil contains information of both the concentration of nutrients in the soil and the moisture content in the soil. It is noted that a soil conductivity X can be the result of a high moisture content and a low concentration of nutrients but also of a low moisture content and a high concentration of nutrients. Combination of soil conductivity measurements with weather data makes it possible to quantify the relative effects of a moisture content increase and a nutrients concentration increase on conductivity. It is noted that the term "operatively connected to the soil" does not mean that it is required to place the soil conductivity sensor in the soil. Soil conductivity sensors using the reflection of high frequency radio waves (reflection of radio waves in the frequency range of 1 MHz to 100 GHz on a ground plane consisting of the soil of interest) expressly make part of the present invention. Also soil conductivity sensors using visible light or hyperspectral imaging expressly makes part of the technology according to the present invention. One way of using hyperspectral imaging to assess the conductivity of the soil is that light absorption and fluorescence by vegetation in the wavelength range between about 400 nm and 900 nm depends on the soil moisture and nutrients content of the soil, especially during periods of drought. By combining these measurements with historical weather data, it is possible to obtain information on individual changes of conductivity and moisture content. Hence, drones equipped with a radio transmitter - receiver combination or a camera and satellite pictures are soil conductivity sensors according to the definition of present invention. This said, it is noted that electrical or capacitive conductivity sensors placed in the soil are preferred since they appear to supply most accurate information. A combination of electrical or capacitive conductivity sensors combined with the beforementioned more indirect measurements are very interesting since the indirect measurements can be used to check if the conductivity measurements provide a good reflection of what happens overall on the soil under investigation.

According to an embodiment of the network, the technology of the present invention consists of at least a water sensor. Preferably, the water sensors comprise a conductivity measurement and spectrophotometric measurements as described in the detailed introduction (see the description of the floaters). The water sensors provide information on nutrients ending up in the surface water and on over-fertilization of the surface water near the agricultural soil under investigation. It is noted that, although previously described as floaters, it is not required to apply floating surface water quality sensors. The sensors may as well be attached to a pole that is driven into the bottom of a ditch or other surface water. Non-floating "floaters" expressly make part of the technology according to the present invention. According to an embodiment of the network, the present invention relates to a physics model that describes the water flowing into the agricultural soil under investigation, the water leaving the agricultural soil under investigation and the water that is retained by the soil. The physics model is required to describe the relation between the nutrients concentration in the soil under investigation and the surface water quality of water bodies in the neighborhood of this soil. The physics model is based on mass balances and geological flow models. The term geological flow model in this patent application is defined as: "computational representation of the subsurface flow of water in geological formations. The flow model makes use of mathematical equations to simulate the physical processes that control fluid movement through the subsurface, such as fluid pressure, rock permeability, and geological structures. It may incorporate data from a variety of sources, including geological maps, well logs, and geophysical surveys, and is used to predict fluid behavior and distribution in subsurface reservoirs."

According to an embodiment of the network, the present invention relates to a physics model describing the water flowing into the surface water body under investigation, the water leaving the surface water body and the water that is retained in the surface water body. It is noted that the physics models describing the water flows in the soil under investigation and the surface water body are interconnected. It is also noted that this interconnection may be very limited i.e., it may very well be the case that a farmer can hardly influence the surface water quality in the ditch or other water bodies close to his agricultural soil. In that particular case it may be very important for the farmer to be able to demonstrate this situation and to prove that he or she is producing according to best practices in spite of low surface water quality near his or her land.

According to an embodiment of the network, the present invention relates to physical and bio(chemical) models describing nutrient mass balances in the soil and surface water bodies under investigation, also comprising the kinetics of biological processes occurring in both the soil and surface waters.

According to an embodiment of the network, the present invention relates to a volatile organic compounds (VOC) measurement in the air above the soil under investigation. VOC measurements provide information on the biological activity in the soil and on the soil surface. This biological activity is related to the TVOC concentration in the soil, which is, as described beforehand, a crucial parameter in soil quality optimization. It is also noted that TVOC is not only related to soil quality in terms of robustness against changes in weather conditions, such as drought or heavy rainfall but also to the effectiveness of the soil to biologically decompose pesticides that were used for crop protection.

Based on the description above, it will be clear for a person skilled in the art of farming that a network of sensors comprising sensors that are operatively connected to the agricultural soil, sensors that are operatively connected to a surface water body near said soil, physics models describing a water balance of the soil, physics models describing a water balance in the surface water body, bio(chemical) models describing the concentration of nutrients and micropollutants, such as pesticides, medicines, microplastics in both soil and surface water body will provide valuable information to a farmer on how to ensure ecologically friendly crop production. In this patent application, ecologically friendly crop production is defined as treating the agricultural soil such that there is no over-fertilization, no underfertilization, a high TVOC concentration in the soil and consequently minimum washout of pesticides and nutrients from soil into surface water.

### Example 1

A farmer A has 5 pinners, each consisting of a soil conductivity sensor, a VOC sensor, a temperature sensor and a air moisture sensor installed on his agricultural land. The farmer also has 3 floaters, consisting of a water conductivity sensor, a temperature sensor and a spectrophotometric sensor installed in the surface water i.e., a large ditch near his land. The beforementioned sensors form a network and upload their measurements to a cloud server real time at a frequency of once per half hour. At the server, data are interpreted by performing model calculations using water balances related to the soil, water balances related to the ditch, nutrient balances comprising (bio)chemical processes in the soil and in the surface water, micropollutant balances comprising (bio)chemical processes in the soil and in the surface water. Also historical and real time data on local weather conditions are used in the models. The models are based on the previously described so called "white box models" that were validated and enriched with so called "black box models" and by results of measuring TVOC indirectly through sensor data fusion. Model validation was performed with help of the farmer who provided properties of his agricultural land and a history file of data describing the status of the land based on chemical soil analysis and use of (green) fertilizer and pesticides on his land.

It appears that the sensor network indicates a relation between the use of fertilizer on the land and surface water quality. Based on the predictions by the sensor network, the farmer reduced the use of fertilizer and found out that the TVOC on his agricultural land did not decrease over time. In other words: the farmer was over-fertilizing his land and now is operating in a more environmentally friendly way.

### Example 2

A farmer B installs pinners and floaters on her land and nearby water body according to example 1. It appears that there is no relation between the water quality in the nearby water body and the concentration of nutrients and TVOC on her land. The total N and P concentrations in the nearby surface water body are structurally too high and in summer there appears to be blue-green algal bloom in the nearby water body. Also, excessive plant growth of duckweed is observed in different parts of the nearby water body. Measurements with the sensor network and model calculations clearly indicate that the surplus of nutrients in the nearby surface water body is not caused by washout of nutrients from the agricultural land of farmer B but by a nutrient source that is several kilometers away from the agricultural land of farmer B. Farmer B can use the measurements with the sensor network to cultivate her land in an optimum way and to prove to the government that she is doing this in an environmentally friendly way. She also can prove that the poor quality of the surface water in the water body on her land is not caused by nutrients from her land ending up in the water body but by a different source in the neighborhood beyond her control. This helps her keep the license to produce.

### Example 3

A farmer C installs pinners and floaters on his land according to example 1. The optimum position of the pinners and floaters is determined by validating the measurements of these floaters and pinners through photos of the land and the nearby surface water, through measurements with a drone taking hyperspectral pictures from the land and the water and through VOC measurements nearby the soil recorded by a drone. After optimizing the location of the pinners and the floaters, farmer C uses the sensor network obtained through this procedure to optimize his crop production.

After elucidating the practical application of the technology according to the present invention, a number of technological add-ons are mentioned that expressly make part of the technology according to the present invention:
- precision wetting of the agricultural land based on measurements with the sensor network.
- precision dosing of pesticides on the agricultural land for crop protection.
- precision application of (green) fertilizer on the land to optimize the TVOC and nutrient concentrations in the soil.
- precision reporting to the government to prove ecologically friendly agriculture and to protect the farmers' license to operate against imprecise model extrapolations applied by the government.
- storing hashes of measurements with the sensor network, applied models and model simulations on a public blockchain to prove immutability of the data.
- applying public water quality data and model simulations outside the borders of the farmers' agricultural land as circumstantial evidence.
- rewarding participants in the sensor network with micropayments through cryptocurrencies for sharing their data with the network.
- enabling parties interested in water quality data and water quality predictions to buy data through micropayments using cryptocurrencies.

Some further examples will be presented that illustrate the working and application of the method(s) according to the present invention.

### Example A

A farmland consisting of sandy soil in a region G with a total surface area of 2.8 ha is surrounded by a large ditch with a characteristic width of 4.5 meter and a average depth of 1.6 meter. The farmland is used by a dairy farm as grazing land for cows.

After inspection of the farmland, 10 soil sensors each equipped with a VOC sensor, a soil impedance sensor, an air temperature sensor and a air moisture sensor were installed in region G. A first subgroup of 5 soil sensors was distributed over the soil surface and operatively connected to the soil at a distance of at least 20 meters from the ditch. A second subgroup of 5 soil sensors was operatively connected to the soil at a distance between 1 and 5 meters from the ditch. The soil sensors were protected against direct contact with cow manure by placing a "small metal wire mesh gate" around each sensor. After inspection of the ditch, it was determined that it was not connected to flowing surface water or any other ditch. In the ditch, 5 water quality sensors were installed by distributing them over the total ditch length at equal distance from each other. Each water quality sensor was equipped with a conductivity sensor, a spectrophotometric sensors measuring the transmittance of the water at 400 nm, 461 nm, 520 nm and 641 nm and a temperature sensor.

During a grow season from March until September, the installed sensors collected soil quality and water quality data at a measuring frequency of once per 15 minutes and uploaded these data to a cloud server where they were stored in a database together with a time stamp. Weather data, comprising rainfall, air moisture, air temperature, number of sun hours, wind direction, wind speed and air pressure specific for region G were stored in the same database, also at a frequency of once per 15 minutes.

Starting from March 1, the momentary soil quality parameters of each soil quality sensor were averaged at a frequency of once per 15 minutes. Subsequently, the standard deviation (sigma) of each momentary soil quality parameter was divided by its average value (av) resulting in coefficients of variation (CV) for each soil quality parameter. In other words, each soil quality parameter i, results in a momentary coefficient of variation CV(i) defined as CV(i)=sigma(i) / av(i). Adding these momentary coefficients of variation CV(i) for the different sensor parameters in each soil sensor S, results in a momentary averaged soil conformity parameter Dm(S). By comparing the values of Dm for all soil sensors, deviations in soil sensor behavior in region G were detected at an early stage. Already after 4 weeks, it was observed that one of the soil quality sensors had Dm values in the range between 5 and 10, which was more than a factor of 3 higher as compared to the other sensors. After careful inspection, it appeared that the VOC sensor in this soil quality sensor was defective so that we disregarded this measurement in further analysis. This intermediate analysis result clearly shows the added value of the momentary averaged soil sensor conformity parameter Dm and the way it is defined.

The soil conformity parameter Dt(S) is a time averaged value of all Dm(S) values over a predefined time window deltat. In other words: deltat = 168 hours means that we obtain a Dt(S) value that is an average of all Dm(S) values that were determined in the time interval deltat of 168 hours i.e., one week. This means that the Dt(S) value is a kind of moving average of Dm(S) values, with in this particular case, a moving time window size of one week. In our test we observed that in June, the Dt value of one of the 5 soil quality sensors near the ditch, started to show an increase of Dm values while the Dt values of this sensor were still in the same range of the other sensors. This result indicates that not a gradual process but a "sudden event" was the cause of the observed sensor behavior deviations. After discussion with the farmer and local inspection it appeared that the farmer had removed dead plant material from the ditch near the place where the soil quality sensor was installed and that he put this dead plant material near the edge of the ditch. As a result, the soil quality sensor detected very high VOC concentrations as well as a decrease in soil impedance (increase in
conductivity). Based on the analysis, the soil quality sensor data of the sensor near the dead plant material were disregarded in the soil and surface water quality analysis. This intermediate analysis result clearly shows the added value of the time averaged soil sensor conformity parameter Dt and the way it is defined.

Mass balances were made to quantify the amount of water added to area G (mainly rainfall), leaving area G (mainly evaporation), water added to the ditch (mainly washout from the soil) and water leaving the ditch (mainly direct evaporation and water use through plant uptake). In other words: from the mass balances, we concluded that region G consists of sandy soil, with hardly any seepage and sufficient rainfall to grow grass without irrigation.

In order the calculate the "local wash out of nutrients from the soil parameter" Pwash we applied following procedure: At periods of rainfall, we calculated the product of total rainfall R [mm] and the time averaged values of soil conductivity C divided by the time averaged concentration of volatile organic compounds in the air measured 15 cm above the soil VOC. It is noted that it is essential to take the time averaged values of these parameters with, for example a moving time window size of one week, and not the momentary averaged values. Especially the VOC values may fluctuate depending on weather conditions and a time frame of a week appears to account for these type of fluctuations. Summarizing, we obtained for region G a parameter Pwash = constant * R * C / VOC.

The concentration of nutrients in the ditch is mainly determined by water supply, water evaporation, Pwash and plant growth. The primary water quality parameter related to the concentration of nutrients in the ditch water, i.e., phosphate, nitrate, ammonium and dissolved organic carbon containing conductive acid groups, is conductivity. Since all 5 water sensors produced similar water conformity parameters Dm and Dt, we averaged the conductivity measurements of the water sensors according to the moving average with a moving time window size of one week. This provides us the nutrient concentration parameter as a function of time N(t).

We analyzed the relation between Pwash and N(T) and found out that an increase of Pwash nearly always results in an increase of N(T) within a time delay of about 2 days. Depending on rainfall, the effect of Pwash on N(T) can last for a long time i.e., for a period of time longer than one week.

From this observation, we concluded that in region G, a significant amount of nutrients washes from the soil into the ditch i.e., into the local surface water. Based on this finding, the farmer decided to decrease the amount of fertilizer that is applied to the soil and to take measures to increase the amount of living total organic carbon LTOC in the soil since LTOC is able to retain nutrients in the soil and prevent them from washing out to the ground water and surface water.

### Example B

During the test as described in example A, we saw an increase of light absorption (decrease
of the transmission values) of the water quality sensors in the ditch. Especially T400 (the transmission of UVA light with a wavelength of 400 nm and T461) decreased after periods of rainfall and this decrease is in phase with N(t). This provides an extra parameter to relate Pwash to surface water quality that can be incorporated in the method(s).

### Example C

This example relates to the test as described in example A. In July and August, the spectroscopic measurements of our water quality sensor appeared to be disturbed by fouling of the transparent tube through which the light is coupled into the water to measure water quality. July and August are also the months that nearly the complete ditch surface was covered with duckweed and that we measured high nutrient concentrations in the water (both through the conductivity sensor and through samples we took and analyzed in a laboratory on nitrate, ammonium, phosphate and TOC).
This result shows that spectrophotometric sensor fouling is a measure for a too high nutrient concentration in the ditch and that we can use a runaway of the spectroscopic sensor signal in terms of Dm as a strong indication for a too high nutrient concentration in the surface water.

### Example D

This example relates to the test as described in example A. During the whole season, photos of the surface water in the ditch were taken at 10 different places in region G. The photos were analyzed on the total water surface that was covered with water plants. The rate at which the water surface is covered with water plants appears to relate to water temperature and the concentration of nutrients in the water. This example shows that pictures of surface water can be used as a water sensor that can be incorporated in the method(s).

### Example E

In this example, the test in example A was repeated with the difference that a strip of 5 meters on either side of the ditch was not fertilized. Additionally, cows were not allowed to graze on the strip. In this case we observed a marked difference between Pwash _strip, determined for the 5 soil sensors installed on the strip near the ditch, and Pwash_grazing _land, determined for the 5 sensors on the grazing land. The time averaged value of Pwash_strip was only 30% of Pwash_grazing_land. Also P _wash_grazing land correlated better with Ntot(t) than P_wash_strip. This result indicates that it makes sense for the farmer to apply a "non fertilize and non grazing strip" of 5 meters from the ditch. By doing this, less nutrients are washed out from the soil into the ditch, resulting in better surface water quality.

### Example F

This example relates to the test in example A. The algorithm for Pwash was improved based on the data collected during the test. The improved algorithm for Pwash was: Pwash = constant1 * R * C / (1 + constant2 * VOCa) with a < 1. This new algorithm resulted in a better correlation between Pwash and N(t). Based on Pwash, the total concentration of nutrients in the ditch N(t), defined as the sum of the nitrate, ammonium, phosphate concentrations, each normalized for their maximum allowable concentration in ditch water, were successfully and automatically predicted on our server using the soil sensor data and weather data as input.

### Example G

This example relates to the test in example A. The algorithm for N(t) was improved by predicting N(t) using following parameters: conductivity C, surface coverage degree of the ditch water with water plants SC in percent, water temperature T, UVA transmission T400. The algorithm was determined by analyzing samples of ditch water at different conditions throughout the grow season.

The improved algorithm for N(t) was: N(t)= constant1 * C * (1+ constant2 *SCb) / (1 + constant3 * Tc + constant4 * T400d), where exponent b > 1, exponent c > 1 and exponent d < 1. The observed dependance of N(t) from SC can be understood from the lack of light falling into the ditch water when water surface is covered by, for example, duckweed. Also, less oxygen is introduced into the water during night in case of a duckweed blanket on the water surface. This results in less biological activity in the ditch water and therefor in higher concentrations of N(t) in the water. The decrease of N(t) with increasing temperature can be understood by an increase of biological activity in the ditch water with increasing temperature. The decrease of N(t) with increasing T400 can be understood from the reasoning that the lower the value of T400, the more organic material is present in the ditch that can be decomposed into nutrients.

The improved algorithm for N(t) resulted in good agreement between analysis results from nutrient samples taken from the ditch and calculated total values of N(t) using the sensor values and the algorithm. At the same time, the correlation between Pwash as defined in example F and the N(t) values calculated with the improved algorithm significantly improved as well. Both the new Pwash and N(t) algorithms were successfully applied to advise farmers on their fertilization strategy.

### Example H

This example relates to the test in example A. In order to obtain a more general algorithm GA, as compared the previous examples, that autotunes its parameters based on real time and inline sensor measurements in region G we applied the following procedure:
1. Install sensors: Install sensors at key locations in the surface water bodies and soil to collect on-line, real-time data of water and soil quality. The sensors can measure various parameters such as pH, temperature, dissolved oxygen, conductivity, turbidity, and various contaminants such as heavy metals and organic pollutants in water, and conductivity, moisture, and VOC concentration in the air above the soil.
2. Data preprocessing: Collect the data from the sensors and preprocess it by removing outliers and errors, imputing missing values, and normalizing the data.
3. Model development: Develop a first-principles model using a nonlinear, reduced-order state-space model comprising mass balances for water and dissolved nutrients, which can provide an accurate representation of the physical and chemical processes that govern surface water quality. Incorporate an AI machine learning algorithm into the model to enhance its predictive power.
4. Data-driven models: Combine the first-principles model with data-driven models such as multiple linear regression, K nearest neighbors, gradient-boosting decision tree, and support vector regression to enhance the accuracy of estimators. These models can capture complex relationships between the input features and the water quality parameters.
5. Soil-water relationship: Analyze the correlation between the soil quality parameters and surface water quality parameters using statistical techniques such as correlation analysis, regression analysis, and principal component analysis. This will provide insights into how soil quality affects water quality.
6. Model evaluation: Evaluate the accuracy of the model by comparing the predicted values with the actual values. Use statistical metrics such as mean absolute error, root mean square error, and coefficient of determination to quantify the performance of the model.
7. Continuous monitoring: Continuously monitor the water and soil quality using the sensors and update the model with the latest data. This will ensure that the model stays up-to-date with the changing conditions in the water and soil.
8. Location selection: Use modern artificial intelligence and geospatial probabilistic risk modelling techniques to choose the locations wisely to monitor water and soil quality. These techniques can identify the areas that are at high risk of pollution and prioritize the monitoring efforts.
9. Decision making: Use the results from the model to make informed decisions about the management of surface water and soil quality. This can include actions such as regulating pollutant discharges, implementing treatment technologies, or taking measures to prevent pollution in both soil and water.

As can be understood from the procedure steps 1 to 9, algorithm GA consists of various building blocks: data preprocessing algorithms, algorithms based on first principle models comprising mass balances for water and nutrients in the water, data driven algorithms, model evaluation algorithms and decision making algorithms.

The algorithms are installed on a server and work iteratively together to minimize real time the difference between model predictions and sensor measurements. The result is an algorithm GA that can be used to predict both soil and surface water qualities in region G.

The present invention is by no means limited to the above-described preferred embodiments. The rights sought are defined by the following claims within the scope of which many modifications can be envisaged.

## Claims

1. Network of sensors for measuring soil and/or surface water quality, the network comprising:
- a controller and/or a processor;
- at least a sensor from a group of soil sensors;
- at least a sensor from a group of water quality sensors measuring at least water conductivity;
- said controller and/or said processor being configured with at least one soil sensor fusion algorithm that is configured for converting soil sensor data of the at least one sensor from the group of soil sensors into a predetermined local wash out of nutrients from the soil parameter (P_{wash}), that is indicative for a quantity of nutrients that is washed from the soil, wherein the soil sensor fusion algorithm comprises combining at least weather data with soil sensor data comprising soil impedance data;
- said controller and/or said processor being configured with at least one water sensor fusion algorithm that is configured for translating the water quality sensor data to a concentration of nutrients in the surface water, wherein the water sensor fusion algorithm is at least based on water temperature and water conductivity as input;
- first means for connecting the sensors to a server and/or second means for connecting the sensors directly or indirectly to a blockchain;
- a server or blockchain receiving data from one or more of the sensors, wherein the server or blockchain is configured for storing individual sensor data in a database, each of the individual sensor data containing time stamps of the data, the sensor data and the GPS coordinates of each sensor; and
- said controller and/or said processor being configured with at least one conformity sensor fusion algorithm that is configured for producing data conformity parameters Dm and Dt for each sensor in at least one region (G), wherein the algorithm is configured to calculate the conformity parameters, momentary averaged soil sensor conformity parameter (Dm) and time averaged soil sensor conformity parameter (Dt), of a sensor of the one or more sensors by providing a measure for the momentary and time averaged deviations from sensor data to all sensor data in region G respectively, wherein the controller and/or processor are configured for performing the data processing and/or calculations for the sensor fusion algorithms using first-principles and/or machine learning models.

2. Network according to claim 1, wherein:
- the at least one sensor from the group soil sensors is a soil moisture sensor that is configured for measuring at least soil resistance between at least two electrodes, air temperature, relative humidity of the air, air pressure and VOC content in the air;
- the at least one water sensor from the group of water quality sensors is a floating water quality sensor that is further configured for measuring at least water temperature, transmission of light through the water at five or more wavelengths in the UVA and/or visible and/or nIR regions;
- the local wash out of nutrients from the soil parameter (P_{wash}) is a "local wash out of nutrients from the soil" parameter (P_{wash});
- the at least one water sensor fusion algorithm is configured to translate the water quality sensor data into a concentration of green algae and / or blue-green algae in the surface water;
- the soil moisture sensors comprise pinners, and wherein the water quality sensors comprise type one floaters and type two floaters;
the network further comprises:
- at least one graphical representation of sensor data on a map with GPS coordinates **characterized by** means to express both the overall surface water quality as well as means to characterize the certainty of the surface water quality assessment;
- a reward system that automatically rewards sensor owners with crypto tokens relative to the amount of data packets their sensors upload to the network of surface water quality sensors.

3. Network according to claim 1 or 2, wherein the individual sensor data stored in the blockchain is a hash of the sensor data received from the sensors and/or the individual sensor data stored by the server.

4. Network according to claim 2 or 3, when dependent on claim 2, wherein both a type one and a type two floating water quality sensor is configured to measure a temperature of the water, electrical conductivity (EC) of the water, and wherein:
- a type one floating water quality sensor is further configured to measure light transmission at preferably 5 wavelengths in the UVA and visible regions and/or near infrared (nIR) regions; and
- a type two floating water quality sensor is further configured to measure light transmission at preferably 7 wavelengths and the fluorescence spectrum resulting from excitation wavelengths at each of these 7 wavelengths in the range between 300 nm and 850 nm,
and wherein the network comprises at least one sensor from the group of type two floating water quality sensors.

5. Network according to any one of the preceding claims, further comprising at least one VOC sensor that is configured to measure a VOC-content in the air, and wherein the at least one VOC sensor is positioned at a predetermined distance from a ground surface and preferably is positioned at a distance of at most 1 meter above the ground surface.

6. Network according to any one of the preceding claims, further comprising one or more sensors configured to measure weather conditions, preferably local weather conditions, and wherein the weather data preferably comprise one or more of air humidity, air pressure; and/or (air) temperature, further preferably comprising a controller and/or a processor, wherein the network, preferably the controller and/or processor, and/or the sensor fusion algorithm, are preferably configured to process weather data and/or weather prediction data.

7. Network according to any one of the preceding claims, wherein the network, preferably one or more of the sensor fusion algorithms therein, is configured to calculate a present and/or future quality of agricultural land expressed in conformity parameters momentary averaged soil sensor conformity parameter (Dm) and time averaged soil sensor conformity parameter (Dt) over time.

8. Method for measuring soil and/or surface water quality, the method comprising the steps of:
- providing a network according to any one of the preceding claims;
- collecting sensor data using the sensors; and
- converting, by the at least first fusion algorithm, soil sensor data of the at least one soil sensor into a predetermined local wash out of nutrients from the soil parameter (P_{wash}) indicative for a quantity of nutrients that is washed from the soil by combining at least weather data with soil sensor data comprising soil impedance data; and
- translating, by the at least second fusion algorithm, the floating water quality sensor data to a concentration of nutrients in the surface water,
further preferably comprising the steps of:
- sending the sensor data to a server and/or blockchain;
- storing the sensor data in the blockchain and/or at the server, preferably as individual sensor data containing time stamps of the data, the sensor data and the GPS coordinates of each sensor,
further preferably comprising the steps of:
- producing, using at least one conformity sensor fusion algorithm, data conformity parameters momentary averaged soil sensor conformity parameter (Dm) and time averaged soil sensor conformity parameter (Dt) for each sensor in at least one region (G),
wherein the producing comprises providing a measure for the momentary and time averaged deviations from sensor data to all sensor data in region (G) respectively.

9. Method for measuring soil and/or surface water quality according to claim 8, wherein the method is a computer-implemented method.

10. A computer program comprising instructions which, when the program is executed by a controller and/or a processor of a network of sensors according to any of the claims 1-7, causes the network of sensors to carry out the steps of:
- collecting soil sensor data and water quality sensor data using the soil sensor and water quality sensor of said network of sensors; and
- converting, by the at least first fusion algorithm, said soil sensor data of the at least one soil sensor into a predetermined local wash out of nutrients from the soil parameter (P_{wash}) indicative for a quantity of nutrients that is washed from the soil by combining at least weather data with soil sensor data comprising soil impedance data; and
- translating, by the at least second fusion algorithm, said water quality sensor data to a concentration of nutrients in the surface water, further comprising the steps of performing the data processing and/or calculations for the sensor fusion algorithms using first-principles and/or machine learning models.

11. Method for realizing a network of surface water quality sensors according to one of the claims 1 - 7, wherein the method preferably comprises the steps of:
- providing at least a sensor from a group of soil sensors;
- providing at least a sensor from a group of water quality sensors measuring at least water conductivity;
- providing at least a first and a second fusion algorithm configured to respectively convert soil sensor data of the at least one soil sensor into a predetermined local wash out of nutrients from the soil parameter (P_{wash}) indicative for a quantity of nutrients that is washed from the soil by combining at least weather data with soil sensor data comprising soil impedance data and translate the floating water quality sensor data to a concentration of nutrients in the surface water;
- positioning the sensors in the soil and/or a surface water in at least a region (G); and
- providing a blockchain and/or a server for storing the sensor data.

12. Method for measuring the robustness of agricultural land against droughts and heavy rains by using a network according to one of the claims 1 - 7.

13. Method for measuring and/or calculating an optimal nutrient concentration in agricultural land by using a network according to one of the claims 1 - 7.

14. Method for determining the presence or absence of a correlation between a quality of agricultural land and the wash-out of nutrients to surface water by using a network according to one of the claims 1 - 7.

15. Use of a network according to any one of the claims 1 - 7 for measuring the robustness of agricultural land against droughts and heavy rains and/or for measuring and/or calculating an optimal nutrient concentration in agricultural land and/or for determining the presence or absence of a correlation between a quality of agricultural land and the wash-out of nutrients to surface water.

## Patentansprüche

1. Sensornetzwerk zur Messung der Boden- und/oder Oberflächenwasserqualität, wobei das Netzwerk umfasst:
- eine Steuereinheit und/oder einen Prozessor;
- mindestens einen Sensor aus einer Gruppe von Bodensensoren;
- mindestens einen Sensor aus einer Gruppe von Wasserqualitätssensoren, die mindestens die Leitfähigkeit des Wassers messen;
- wobei die Steuereinheit und/oder der Prozessor mit mindestens einem Boden-Sensorfusionsalgorithmus konfiguriert ist, der dazu eingerichtet ist, Bodensensordaten des mindestens einen Sensors aus der Gruppe der Bodensensoren in einen vorbestimmten Parameter für den lokalen Austrag von Nährstoffen aus dem Boden (Pwash) umzuwandeln, der für eine Menge an Nährstoffen indikativ ist, die aus dem Boden ausgewaschen wird, wobei der Boden-Sensorfusionsalgorithmus das Kombinieren mindestens von Wetterdaten mit Bodensensordaten umfasst, die Bodenimpedanzdaten umfassen;
- wobei die Steuereinheit und/oder der Prozessor mit mindestens einem Wasser-Sensorfusionsalgorithmus konfiguriert ist, der dazu eingerichtet ist, die Wasserqualitätssensordaten in eine Konzentration von Nährstoffen im Oberflächenwasser zu übersetzen, wobei der Wasser-Sensorfusionsalgorithmus mindestens auf Wassertemperatur und Wasserleitfähigkeit als Eingabe basiert;
- erste Mittel zum Verbinden der Sensoren mit einem Server und/oder zweite Mittel zum direkten oder indirekten Verbinden der Sensoren mit einer Blockchain;
- ein Server oder eine Blockchain, der bzw. die Daten von einem oder mehreren der Sensoren empfängt, wobei der Server oder die Blockchain dazu eingerichtet ist, einzelne Sensordaten in einer Datenbank zu speichern, wobei jede der einzelnen Sensordaten Zeitstempel der Daten, die Sensordaten und die GPS-Koordinaten jedes Sensors enthält; und
- wobei die Steuereinheit und/oder der Prozessor mit mindestens einem Konformitäts-Sensorfusionsalgorithmus konfiguriert ist, der dazu eingerichtet ist, Datenkonformitätsparameter Dm und Dt für jeden Sensor in mindestens einer Region (G) zu erzeugen, wobei der Algorithmus dazu eingerichtet ist, die Konformitätsparameter, momentaner gemittelter Boden-Sensorkonformitätsparameter (Dm) und zeitlich gemittelter Boden-Sensorkonformitätsparameter (Dt), eines Sensors der einen oder mehreren Sensoren zu berechnen, indem ein Maß für die momentanen und zeitlich gemittelten Abweichungen der Sensordaten von allen Sensordaten in der Region G jeweils bereitgestellt wird, wobei die Steuereinheit und/oder der Prozessor dazu eingerichtet sind, die Datenverarbeitung und/oder Berechnungen für die Sensorfusionsalgorithmen unter Verwendung von First-Principles- und/oder Machine-Learning-Modellen durchzuführen.

2. Netzwerk nach Anspruch 1, wobei:
- der mindestens eine Sensor aus der Gruppe der Bodensensoren ein Bodenfeuchtesensor ist, der dazu eingerichtet ist, mindestens den Bodenwiderstand zwischen mindestens zwei Elektroden, die Lufttemperatur, die relative Luftfeuchtigkeit, den Luftdruck und den VOC-Gehalt in der Luft zu messen;
- der mindestens eine Sensor aus der Gruppe der Wasserqualitätssensoren ein schwimmender Wasserqualitätssensor ist, der ferner dazu eingerichtet ist, mindestens die Wassertemperatur und die Transmission von Licht durch das Wasser bei fünf oder mehr Wellenlängen im UVA- und/oder sichtbaren und/oder nIR-Bereich zu messen;
- der Parameter für den lokalen Austrag von Nährstoffen aus dem Boden (Pwash) ein Parameter "lokaler Austrag von Nährstoffen aus dem Boden" (Pwash) ist;
- der mindestens eine Wasser-Sensorfusionsalgorithmus dazu eingerichtet ist, die Wasserqualitätssensordaten in eine Konzentration von Grünalgen und/oder Blaualgen im Oberflächenwasser zu übersetzen;
- die Bodenfeuchtesensoren Pinner umfassen, und wobei die Wasserqualitätssensoren Schwimmer vom Typ eins und Schwimmer vom Typ zwei umfassen;
das Netzwerk ferner umfasst:
- mindestens eine grafische Darstellung von Sensordaten auf einer Karte mit GPS-Koordinaten, **gekennzeichnet durch** Mittel zum Ausdrücken sowohl der gesamten Oberflächenwasserqualität als auch Mittel zur Charakterisierung der Sicherheit der Bewertung der Oberflächenwasserqualität;
- ein Belohnungssystem, das Sensorbesitzer automatisch mit Krypto-Token im Verhältnis zur Anzahl der Datenpakete belohnt, die ihre Sensoren in das Netzwerk von Oberflächenwasserqualitätssensoren hochladen.

3. Netzwerk nach Anspruch 1 oder 2, wobei die in der Blockchain gespeicherten einzelnen Sensordaten ein Hash der von den Sensoren empfangenen Sensordaten und/oder der vom Server gespeicherten einzelnen Sensordaten sind.

4. Netzwerk nach Anspruch 2 oder 3, wenn abhängig von Anspruch 2, wobei sowohl ein schwimmender Wasserqualitätssensor vom Typ eins als auch vom Typ zwei dazu eingerichtet ist, eine Temperatur des Wassers und die elektrische Leitfähigkeit (EC) des Wassers zu messen, und wobei:
- ein schwimmender Wasserqualitätssensor vom Typ eins ferner dazu eingerichtet ist, die Lichttransmission vorzugsweise bei 5 Wellenlängen im UVA- und sichtbaren Bereich und/oder im Nahinfrarot-(nIR)-Bereich zu messen; und
- ein schwimmender Wasserqualitätssensor vom Typ zwei ferner dazu eingerichtet ist, die Lichttransmission vorzugsweise bei 7 Wellenlängen sowie das Fluoreszenzspektrum zu messen, das aus Anregungswellenlängen bei jeder dieser 7 Wellenlängen im Bereich zwischen 300 nm und 850 nm resultiert,
und wobei das Netzwerk mindestens einen Sensor aus der Gruppe der schwimmenden Wasserqualitätssensoren vom Typ zwei umfasst.

5. Netzwerk nach einem der vorhergehenden Ansprüche, ferner umfassend mindestens einen VOC-Sensor, der dazu eingerichtet ist, einen VOC-Gehalt in der Luft zu messen, und wobei der mindestens eine VOC-Sensor in einem vorbestimmten Abstand von einer Bodenoberfläche positioniert ist und vorzugsweise in einem Abstand von höchstens 1 Meter über der Bodenoberfläche positioniert ist.

6. Netzwerk nach einem der vorhergehenden Ansprüche, ferner umfassend einen oder mehrere Sensoren, die dazu eingerichtet sind, Wetterbedingungen, vorzugsweise lokale Wetterbedingungen, zu messen, wobei die Wetterdaten vorzugsweise einen oder mehrere der Parameter Luftfeuchtigkeit, Luftdruck und/oder (Luft-)Temperatur umfassen, ferner vorzugsweise eine Steuereinheit und/oder einen Prozessor umfassend, wobei das Netzwerk, vorzugsweise die Steuereinheit und/oder der Prozessor und/oder der Sensorfusionsalgorithmus, vorzugsweise dazu eingerichtet ist, Wetterdaten und/oder Wettervorhersagedaten zu verarbeiten.

7. Netzwerk nach einem der vorhergehenden Ansprüche, wobei das Netzwerk, vorzugsweise einer oder mehrere der darin enthaltenen Sensorfusionsalgorithmen, dazu eingerichtet ist, eine gegenwärtige und/oder zukünftige Qualität von landwirtschaftlichem Land berechnet als Konformitätsparameter momentaner gemittelter Boden-Sensorkonformitätsparameter (Dm) und zeitlich gemittelter Boden-Sensorkonformitätsparameter (Dt) über die Zeit.

8. Verfahren zur Messung der Boden- und/oder Oberflächenwasserqualität, wobei das Verfahren die Schritte umfasst:
- Bereitstellen eines Netzwerks nach einem der vorhergehenden Ansprüche;
- Sammeln von Sensordaten mittels der Sensoren; und
- Umwandeln, durch den mindestens ersten Fusionsalgorithmus, von Bodensensordaten des mindestens einen Bodensensors in einen vorbestimmten Parameter für den lokalen Austrag von Nährstoffen aus dem Boden (Pwash), der für eine Menge an Nährstoffen indikativ ist, die durch Kombinieren mindestens von Wetterdaten mit Bodensensordaten, die Bodenimpedanzdaten umfassen, aus dem Boden ausgewaschen wird; und
- Übersetzen, durch den mindestens zweiten Fusionsalgorithmus, der schwimmenden Wasserqualitätssensordaten in eine Konzentration von Nährstoffen im Oberflächenwasser,
ferner vorzugsweise umfassend die Schritte:
- Senden der Sensordaten an einen Server und/oder eine Blockchain;
- Speichern der Sensordaten in der Blockchain und/oder auf dem Server, vorzugsweise als einzelne Sensordaten enthaltend Zeitstempel der Daten, die Sensordaten und die GPS-Koordinaten jedes Sensors,
ferner vorzugsweise umfassend die Schritte:
- Erzeugen, unter Verwendung mindestens eines Konformitäts-Sensorfusionsalgorithmus, von Datenkonformitätsparametern momentaner gemittelter Boden-Sensorkonformitätsparameter (Dm) und zeitlich gemittelter Boden-Sensorkonformitätsparameter (Dt) für jeden Sensor in mindestens einer Region (G),
wobei das Erzeugen das Bereitstellen eines Maßes für die momentanen und zeitlich gemittelten Abweichungen der Sensordaten von allen Sensordaten in der Region (G) jeweils umfasst.

9. Verfahren zur Messung der Boden- und/oder Oberflächenwasserqualität nach Anspruch 8, wobei das Verfahren ein computerimplementiertes Verfahren ist.

10. Computerprogramm umfassend Anweisungen, die, wenn das Programm von einer Steuereinheit und/oder einem Prozessor eines Sensornetzwerks nach einem der Ansprüche 1-7 ausgeführt wird, bewirken, dass das Sensornetzwerk die Schritte ausführt:
- Sammeln von Bodensensordaten und Wasserqualitätssensordaten mittels des Boden- und Wasserqualitätssensors des genannten Sensornetzwerks; und
- Umwandeln, durch den mindestens ersten Fusionsalgorithmus, der genannten Bodensensordaten des mindestens einen Bodensensors in einen vorbestimmten Parameter für den lokalen Austrag von Nährstoffen aus dem Boden (Pwash), der für eine Menge an Nährstoffen indikativ ist, die aus dem Boden ausgewaschen wird, durch Kombinieren mindestens von Wetterdaten mit Bodensensordaten, die Bodenimpedanzdaten umfassen; und
- Übersetzen, durch den mindestens zweiten Fusionsalgorithmus, der genannten Wasserqualitätssensordaten in eine Konzentration von Nährstoffen im Oberflächenwasser, ferner umfassend die Schritte des Durchführens der Datenverarbeitung und/oder Berechnungen für die Sensorfusionsalgorithmen unter Verwendung von First-Principles- und/oder Machine-Learning-Modellen durchzuführen.

11. Verfahren zur Realisierung eines Netzwerks von Oberflächenwasserqualitätssensoren nach einem der Ansprüche 1-7, wobei das Verfahren vorzugsweise die Schritte umfasst:
- Bereitstellen mindestens eines Sensors aus einer Gruppe von Bodensensoren;
- Bereitstellen mindestens eines Sensors aus einer Gruppe von Wasserqualitätssensoren, die mindestens die Wasserleitfähigkeit messen;
- Bereitstellen mindestens eines ersten und eines zweiten Fusionsalgorithmus, die jeweils dazu eingerichtet sind, Bodensensordaten des mindestens einen Bodensensors in einen vorbestimmten Parameter für den lokalen Austrag von Nährstoffen aus dem Boden (Pwash) umzuwandeln, der für eine Menge an Nährstoffen indikativ ist, die aus dem Boden ausgewaschen wird, indem mindestens Wetterdaten mit Bodensensordaten kombiniert werden, die Bodenimpedanzdaten umfassen, und die schwimmenden Wasserqualitätssensordaten in eine Konzentration von Nährstoffen im Oberflächenwasser zu übersetzen;
- Positionieren der Sensoren im Boden und/oder Oberflächenwasser in mindestens einer Region (G); und
- Bereitstellen einer Blockchain und/oder eines Servers zum Speichern der Sensordaten.

12. Verfahren zur Messung der Robustheit von landwirtschaftlichem Land gegen Dürren und starke Regenfälle unter Verwendung eines Netzwerks nach einem der Ansprüche 1-7.

13. Verfahren zur Messung und/oder Berechnung einer optimalen Nährstoffkonzentration in landwirtschaftlichem Land unter Verwendung eines Netzwerks nach einem der Ansprüche 1-7.

14. Verfahren zur Bestimmung des Vorhandenseins oder Nichtvorhandenseins einer Korrelation zwischen einer Qualität von landwirtschaftlichem Land und dem Austrag von Nährstoffen in Oberflächenwasser unter Verwendung eines Netzwerks nach einem der Ansprüche 1-7.

15. Verwendung eines Netzwerks nach einem der Ansprüche 1-7 zur Messung der Robustheit von landwirtschaftlichem Land gegen Dürren und starke Regenfälle und/oder zur Messung und/oder Berechnung einer optimalen Nährstoffkonzentration in landwirtschaftlichem Land und/oder zur Bestimmung des Vorhandenseins oder Nichtvorhandenseins einer Korrelation zwischen einer Qualität von landwirtschaftlichem Land und dem Austrag von Nährstoffen in Oberflächenwasser.

## Revendications

1. Réseau de capteurs destiné à mesurer la qualité du sol et/ou des eaux de surface, le réseau comprenant :
- un contrôleur et/ou un processeur ;
- au moins un capteur d'un groupe de capteurs de sol;
- au moins un capteur d'un groupe de capteurs de qualité de l'eau mesurant au moins la conductivité de l'eau ;
- ledit contrôleur et/ou ledit processeur étant configuré avec au moins un algorithme de fusion de capteurs de sol qui est configuré pour convertir des données de capteurs de sol du au moins un capteur du groupe de capteurs de sol en un paramètre prédéterminé de lessivage local des nutriments du sol (Pwash), indicatif d'une quantité de nutriments lessivés du sol, l'algorithme de fusion de capteurs de sol comprenant la combinaison d'au moins des données météorologiques avec des données de capteurs de sol comprenant des données d'impédance du sol;
- ledit contrôleur et/ou ledit processeur étant configuré avec au moins un algorithme de fusion de capteurs d'eau configuré pour traduire les données des capteurs de qualité de l'eau en une concentration de nutriments dans l'eau de surface, l'algorithme de fusion de capteurs d'eau étant au moins basé sur la température de l'eau et la conductivité de l'eau comme entrées ;
- des premiers moyens pour connecter les capteurs à un serveur et/ou des deuxièmes moyens pour connecter les capteurs directement ou indirectement à une blockchain ;
- un serveur ou une blockchain recevant des données d'un ou plusieurs capteurs, le serveur ou la blockchain étant configuré pour stocker des données individuelles de capteurs dans une base de données, chacune des données individuelles de capteurs contenant des horodatages des données, les données de capteurs et les coordonnées GPS de chaque capteur ; et
- ledit contrôleur et/ou ledit processeur étant configuré avec au moins un algorithme de fusion de capteurs de conformité configuré pour produire des paramètres de conformité des données Dm et Dt pour chaque capteur dans au moins une région (G), l'algorithme étant configuré pour calculer les paramètres de conformité, paramètre de conformité instantané moyen des capteurs de sol (Dm) et paramètre de conformité moyen temporel des capteurs de sol (Dt), d'un capteur desdits un ou plusieurs capteurs en fournissant une mesure des écarts instantanés et temporellement moyens des données de capteurs par rapport à toutes les données de capteurs dans la région G respectivement, le contrôleur et/ou le processeur étant configuré pour effectuer le traitement des données et/ou les calculs pour les algorithmes de fusion de capteurs en utilisant des modèles de premiers principes et/ou d'apprentissage automatique.

2. Réseau selon la revendication 1, dans lequel :
- au moins un capteur du groupe de capteurs de sol est un capteur d'humidité du sol configuré pour mesurer au moins la résistance du sol entre au moins deux électrodes, la température de l'air, l'humidité relative de l'air, la pression de l'air et la teneur en COV dans l'air ;
- au moins un capteur d'eau du groupe de capteurs de qualité de l'eau est un capteur flottant de qualité de l'eau configuré en outre pour mesurer au moins la température de l'eau et la transmission de la lumière à travers l'eau à cinq longueurs d'onde ou plus dans les domaines UVA et/ou visible et/ou nIR ;
- le paramètre de lessivage local des nutriments du sol (Pwash) est un paramètre "lessivage local des nutriments du sol" (Pwash) ;
- au moins un algorithme de fusion de capteurs d'eau est configuré pour traduire les données des capteurs de qualité de l'eau en une concentration d'algues vertes et/ou d'algues bleu-vert dans l'eau de surface ;
- les capteurs d'humidité du sol comprennent des Pinner, et les capteurs de qualité de l'eau comprennent des flotteurs de type un et des flotteurs de type deux ;
le réseau comprend en outre :
- au moins une représentation graphique des données de capteurs sur une carte avec des coordonnées GPS **caractérisée par** des moyens permettant d'exprimer à la fois la qualité globale des eaux de surface ainsi que des moyens permettant de caractériser la certitude de l'évaluation de la qualité des eaux de surface ;
- un système de récompense qui récompense automatiquement les propriétaires de capteurs avec des jetons cryptographiques proportionnellement à la quantité de paquets de données que leurs capteurs téléchargent vers le réseau de capteurs de qualité des eaux de surface.

3. Réseau selon la revendication 1 ou 2, dans lequel les données individuelles de capteurs stockées dans la blockchain sont un hachage des données de capteurs reçues des capteurs et/ou des données individuelles de capteurs stockées par le serveur.

4. Réseau selon la revendication 2 ou 3, lorsqu'il dépend de la revendication 2, dans lequel un capteur flottant de qualité de l'eau de type un et un capteur flottant de qualité de l'eau de type deux sont configurés pour mesurer une température de l'eau et la conductivité électrique (EC) de l'eau, et dans lequel :
- un capteur flottant de qualité de l'eau de type un est configuré en outre pour mesurer la transmission de la lumière de préférence à 5 longueurs d'onde dans les domaines UVA et visible et/ou proche infrarouge (nIR) ; et
- un capteur flottant de qualité de l'eau de type deux est configuré en outre pour mesurer la transmission de la lumière de préférence à 7 longueurs d'onde et le spectre de fluorescence résultant des longueurs d'onde d'excitation à chacune de ces 7 longueurs d'onde dans la plage comprise entre 300 nm et 850 nm,
et dans lequel le réseau comprend au moins un capteur du groupe de capteurs flottants de qualité de l'eau de type deux.

5. Réseau selon l'une quelconque des revendications précédentes, comprenant en outre au moins un capteur de COV configuré pour mesurer une teneur en COV dans l'air, et dans lequel au moins un capteur de COV est positionné à une distance prédéterminée d'une surface du sol et est de préférence positionné à une distance d'au plus 1 mètre au-dessus de la surface du sol.

6. Réseau selon l'une quelconque des revendications précédentes, comprenant en outre un ou plusieurs capteurs configurés pour mesurer des conditions météorologiques, de préférence des conditions météorologiques locales, et dans lequel les données météorologiques comprennent de préférence un ou plusieurs des paramètres suivants : humidité de l'air, pression de l'air et/ou température de l'air, comprenant en outre de préférence un contrôleur et/ou un processeur, dans lequel le réseau, de préférence le contrôleur et/ou le processeur et/ou l'algorithme de fusion de capteurs, est configuré pour traiter des données météorologiques et/ou des données de prédiction météorologique.

7. Réseau selon l'une quelconque des revendications précédentes, dans lequel le réseau, de préférence un ou plusieurs des algorithmes de fusion de capteurs qu'il contient, est configuré pour calculer une qualité présente et/ou future des terres agricoles exprimée en paramètres de conformité paramètre de conformité instantané moyen des capteurs de sol (Dm) et paramètre de conformité moyen temporel des capteurs de sol (Dt) au cours du temps.

8. Procédé de mesure de la qualité du sol et/ou des eaux de surface, le procédé comprenant les étapes de :
- fournir un réseau selon l'une quelconque des revendications précédentes ;
- collecter des données de capteurs à l'aide des capteurs ; et
- convertir, par au moins un premier algorithme de fusion, des données de capteurs de sol du au moins un capteur de sol en un paramètre prédéterminé de lessivage local des nutriments du sol (Pwash) indicatif d'une quantité de nutriments lessivés du sol en combinant au moins des données météorologiques avec des données de capteurs de sol comprenant des données d'impédance du sol ; et
- traduire, par au moins un second algorithme de fusion, les données du capteur flottant de qualité de l'eau en une concentration de nutriments dans l'eau de surface,
comprenant en outre de préférence les étapes de :
- envoyer les données de capteurs à un serveur et/ou à une blockchain ;
- stocker les données de capteurs dans la blockchain et/ou sur le serveur, de préférence en tant que données individuelles de capteurs contenant des horodatages des données, les données de capteurs et les coordonnées GPS de chaque capteur,
comprenant en outre de préférence les étapes de :
- produire, à l'aide d'au moins un algorithme de fusion de capteurs de conformité, des paramètres de conformité des données paramètre de conformité instantané moyen des capteurs de sol (Dm) et paramètre de conformité moyen temporel des capteurs de sol (Dt) pour chaque capteur dans au moins une région (G),
dans lequel la production comprend la fourniture d'une mesure des écarts instantanés et temporellement moyens des données de capteurs par rapport à toutes les données de capteurs dans la région (G) respectivement.

9. Procédé de mesure de la qualité du sol et/ou des eaux de surface selon la revendication 8, dans lequel le procédé est un procédé mis en œuvre par ordinateur.

10. Programme informatique comprenant des instructions qui, lorsque le programme est exécuté par un contrôleur et/ou un processeur d'un réseau de capteurs selon l'une quelconque des revendications 1 à 7, amène le réseau de capteurs à effectuer les étapes de :
- collecter des données de capteurs de sol et des données de capteurs de qualité de l'eau à l'aide du capteur de sol et du capteur de qualité de l'eau dudit réseau de capteurs ; et
- convertir, par au moins un premier algorithme de fusion, lesdites données de capteurs de sol du au moins un capteur de sol en un paramètre prédéterminé de lessivage local des nutriments du sol (Pwash) indicatif d'une quantité de nutriments lessivés du sol en combinant au moins des données météorologiques avec des données de capteurs de sol comprenant des données d'impédance du sol; et
- traduire, par au moins un second algorithme de fusion, lesdites données de capteurs de qualité de l'eau en une concentration de nutriments dans l'eau de surface, comprenant en outre les étapes d'effectuer le traitement des données et/ou les calculs pour les algorithmes de fusion de capteurs en utilisant des modèles de premiers principes et/ou d'apprentissage automatique.

11. Procédé de réalisation d'un réseau de capteurs de qualité des eaux de surface selon l'une quelconque des revendications 1 à 7, le procédé comprenant de préférence les étapes de :
- fournir au moins un capteur d'un groupe de capteurs de sol;
- fournir au moins un capteur d'un groupe de capteurs de qualité de l'eau mesurant au moins la conductivité de l'eau;
- fournir au moins un premier et un second algorithme de fusion configurés pour convertir respectivement des données de capteurs de sol du au moins un capteur de sol en un paramètre prédéterminé de lessivage local des nutriments du sol (Pwash) indicatif d'une quantité de nutriments lessivés du sol en combinant au moins des données météorologiques avec des données de capteurs de sol comprenant des données d'impédance du sol et traduire les données du capteur flottant de qualité de l'eau en une concentration de nutriments dans l'eau de surface ;
- positionner les capteurs dans le sol et/ou dans les eaux de surface dans au moins une région (G) ; et
- fournir une blockchain et/ou un serveur pour stocker les données de capteurs.

12. Procédé de mesure de la robustesse des terres agricoles face aux sécheresses et aux fortes pluies en utilisant un réseau selon l'une quelconque des revendications 1 à 7.

13. Procédé de mesure et/ou de calcul d'une concentration optimale de nutriments dans les terres agricoles en utilisant un réseau selon l'une quelconque des revendications 1 à 7.

14. Procédé de détermination de la présence ou de l'absence d'une corrélation entre une qualité des terres agricoles et le lessivage des nutriments vers les eaux de surface en utilisant un réseau selon l'une quelconque des revendications 1 à 7.

15. Utilisation d'un réseau selon l'une quelconque des revendications 1 à 7 pour mesurer la robustesse des terres agricoles face aux sécheresses et aux fortes pluies et/ou pour mesurer et/ou calculer une concentration optimale de nutriments dans les terres agricoles et/ou pour déterminer la présence ou l'absence d'une corrélation entre une qualité des terres agricoles et le lessivage des nutriments vers les eaux de surface.
